# DEMANDE DE BREVET EUROPEEN

(11) **EP 3 603 490 A1**
(43) Date de publication de la demande: **05.02.2020**
(21) Numéro de dépôt: 18306067.2
(22) Date de dépôt: 03.08.2018
(51) Int. Cl.: A61B 5/00, A61B 5/145

(54) **RATIO MÉTAL/SURFACE DE MICROAIGUILLE**

(71) Demandeur: PKvitality, 75001 Paris (FR)
(72) Inventeur: PIERART, Luc, 94800 VILLEJUIF (FR)
(74) Mandataire: Regimbeau

(57) **Abrégé**

L'invention propose un capteur pour mesurer des analytes présentes dans un fluide corporel dans un être vivant, le capteur comprend :
- un substrat (242),
- au moins une micro-aiguille (210), la micro-aiguille (210) comprenant :
une base (210a), solidaire du substrat (242), et comprenant en partie un revêtement métallique en forme de piste (210c),
une pointe (210b), solidaire de la base (210a), entièrement recouverte d'un revêtement métallique,
un élément biochimique recouvrant la pointe (210b), apte à réagir avec des analytes et qui recouvre au moins partiellement le revêtement métallique de la microaiguille (210),
caractérisé en ce que le ratio surface métallique de la base (210a) sur surface totale de la base (210a) est compris entre 1 et 20%.

## Description

### DOMAINE TECHNIQUE GENERAL

La présente invention concerne un dispositif de surveillance corporelle via analyse de liquide corporel, typiquement interstitiel, à l'aide de microaiguilles.

Plus précisément, la présente invention concerne la gestion de la sueur présente sur la peau là où sont positionnées les microaiguilles.

### ETAT DE L'ART

Certaines pathologies comme le diabète nécessitent une surveillance quotidienne de paramètres biochimiques du corps humain, i.e. des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

Toutefois, lorsqu'un tel dispositif comporte des microaiguilles, il peut exister des interférences liées à la sueur présente sur la peau. Ces interférences dépendent en outre de l'épaisseur de l'épiderme, qui est variable selon les individus : l'épaisseur de peau pour atteindre le liquide interstitiel n'est alors pas la même.

Pour résoudre cela, il existe des solutions visant à masquer certaines parties des microaiguilles, pour éviter qu'elles n'interfèrent. On peut citer un revêtement isolant, comme dans le document US 2014/0275897 par exemple. Toutefois, ces produits multicouches sont coûteux à réaliser et difficile à produire.

L'invention vise à adresser ces difficultés.

### PRESENTATION DE L'INVENTION

Pour pallier à certaines de ces difficultés, l'invention propose un capteur pour mesurer des analytes présentes dans un fluide corporel dans un être vivant, le capteur comprend :
- un substrat,
- au moins une micro-aiguille, la micro-aiguille comprenant :
   une base, solidaire du substrat, et comprenant en partie un revêtement métallique en forme de piste,
   une pointe, solidaire de la base, entièrement recouverte d'un revêtement métallique,
   un élément biochimique recouvrant la pointe, apte à réagir avec des analytes et qui recouvre au moins partiellement le revêtement métallique de la microaiguille,
caractérisé en ce que le ratio surface métallique de la base sur surface totale de la base est compris entre 1 et 20%,

Dans un mode de réalisation, la hauteur de la base est supérieure à 140 µm, préférablement supérieure à 200µm.

Dans un mode de réalisation, le capteur comprend une pluralité de groupes comprenant au moins une microaiguille telle que définie précédemment un groupe définissant une électrode de travail, un groupe définissant une électrode de référence, et préférablement une contre-électrode, voire une électrode neutre.

Dans un mode de réalisation, les microaiguilles des deux groupes sont écartées entre elles d'au moins 1 mm (pour éviter la pollution par fabrication de peroxyde d'hydrogène).

Dans un mode de réalisation, la hauteur de la pointe est comprise entre 200 et 700 µm, préférablement entre 300 et 500 µm.

Dans un mode de réalisation, la base a une forme pyramidale.

Dans un mode de réalisation, la base a une forme pyramidale, avec une largeur supérieure à 140 µm.

Dans un mode de réalisation, le substrat est en polymère ou silicium, ou pas en métal.

Dans un mode de réalisation, l'élément biochimique recouvre intégralement le revêtement métallique de la pointe et partiellement le revêtement métallique de la base.

L'invention propose aussi un système de surveillance corporelle destiné à être attaché à un membre d'un être vivant, comprenant :
- un boitier, comprenant une face de couplage,
- une capsule comprenant un capteur tel que décrit précédemment, la capsule pouvant s'engager de façon amovible avec la face de couplage du boitier.

Dans un mode de réalisation, le système comprend en outre un patch attachable à la capsule, le patch ayant un pouvoir adhésif à la peau.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 illustre en vue éclatée un bracelet/lanière, un boitier, une capsule et un patch tel qu'utilisable dans le cadre de l'invention,
- La figure 2 illustre une vue illustrative de plusieurs microaiguilles différentes, sans respect des proportions ou des angles.

### DESCRIPTION DETAILLEE

### Capteur

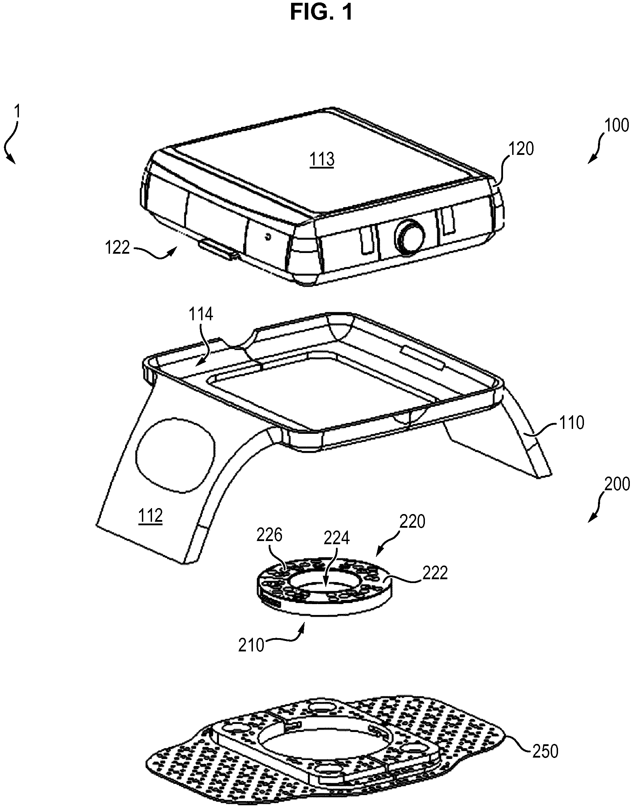

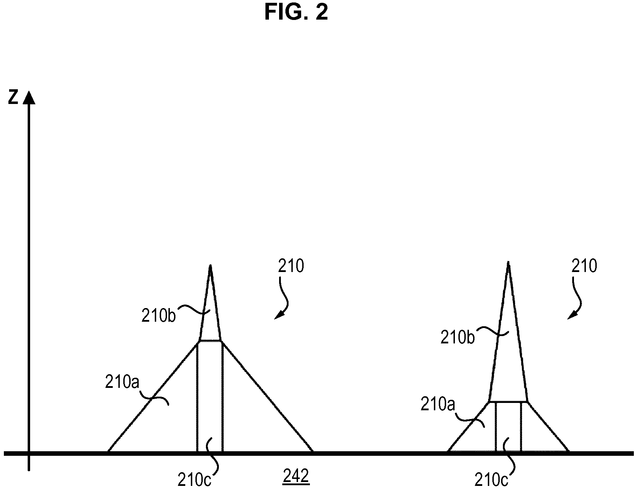

En relation avec les **figures 1** **et** **2****,** un capteur pour mesurer des analytes présentes dans un fluide corporel d'un être vivant (typiquement le liquide interstitiel) va être décrit. Ce capteur s'insère dans un système de surveillance corporelle 1 qui sera décrit par la suite.

Le capteur comprend un substrat et au moins une microaiguille 210, préférablement un plusieurs ensembles de microaiguilles 210.

Par microaiguilles 210, on entend une aiguille préférablement pleine (car pas besoin de prélever de liquide), d'une hauteur totale comprise entre 100µm et 1000µm, préférablement 300µm et 800µm. Chacune de ces caractéristiques avantageuses des microaiguilles 210 peut être prise séparément ou en combinaison avec les autres.

La micro-aiguille 210 comprend une base 210a, solidaire du substrat 242 et qui s'en étend selon une direction principale Z. La microaiguille 210 comprend en outre une pointe 210b, solidaire de la base 210a, qui s'étend aussi selon la direction principale Z. Visuellement, il peut y avoir un changement de pente entre la base 210a et la pointe 210b. On a donc substrat 242, puis base 210a, puis pointe 210b.

La pointe 210b est entièrement recouverte d'un revêtement métallique.

La base 210a est partiellement recouverte d'un revêtement métallique, sous forme de piste 210c depuis le substrat 242 jusqu'au revêtement métallique de la pointe 210b.

Enfin, un élément biochimique, qui est apte à réagir avec l'analyte (pour permettre des mesures relatives à cet analyte) recouvre au moins partiellement le revêtement métallique. Préférablement, l'intégralité du revêtement métallique de la pointe 210b est recouverte de l'élément biochimique et le revêtement métallique de la base 210a n'est pas recouverte. Toutefois, à cause des procédés de fabrication, il se peut qu'une partie du revêtement métallique de la base 210a soit aussi recouvert de l'élément biochimique. Or, la base 210a peut être au contact de la sueur de la peau via le percement de la peau.

Dans les procédés utilisés, l'élément biochimique se dépose uniquement sur le revêtement métallique et non pas sur le substrat 242.

A cet égard, le substrat 242 est en polymère ou en silicium, voire tout autre matériau non-métallique. La base 210a et la pointe 210b (avant revêtement métallique) peuvent être réalisées dans le même matériau que le substrat.

Le métal a aussi pour fonction de transmettre les signaux électriques générés par l'élément biochimique lorsqu'il réagit avec l'analyte.

Afin de limiter l'influence de la sueur, la solution proposée est de limiter la présence de l'élément biochimique sur la base 210a, ce qui signifie qu'il faut limiter la présence du revêtement métallique sur la base. Pour cela, le ratio de surface du revêtement métallique de la base 210a sur la surface totale du la base 210a est compris entre 1 et 20%. Cela signifie que le revêtement métallique sous forme de piste 210c de la base 210a représente entre 1 et 20% de la surface de la base 210a.

La valeur de 1% est lié au fait que la présence de revêtement métallique sur la base 210a est nécessaire pour la transmission des signaux et la valeur de 20% est liée au fait de limiter le risque d'avoir de l'élément biochimique sur la base 210a de la microaiguille 210.

Préférablement, le ratio est compris entre 1% et 15%.

Par « surface », on entend la surface exposée à l'environnement externe.

La base 210a a une dimension selon la direction principale Z (appelée hauteur) supérieure à 140µm, et préférablement supérieure à 200 µm. Cette valeur est liée à l'épaisseur de l'épiderme, qui est généralement comprise entre 80 et 180µm avant d'accéder au liquide interstitiel (selon le type de peau). Avec 200µm, on augmente fortement la probabilité que la pointe 210b soit bien baignée de liquide interstitiel.

La pointe 210b, quant à elle, a une dimension selon la direction principale (appelée hauteur) comprise entre 200 et 700 µm et préférablement comprise entre 300 et 500 µm.

Au total, selon les modes de réalisation, la microaiguille 210 a une hauteur minimale de 340µm et une hauteur maximale environ égale à 1000µm. La hauteur de la pointe 210b et la hauteur de la base 210c sont choisies pour s'assurer d'une part que la pointe soit bien au contact du liquide interstitiel et d'autre part que l'hypoderme (où se trouve les nerfs mécaniques et une plus grande densité de vaisseau sanguin) ne soit pas atteint.

Le capteur comprend typiquement plusieurs groupes de microaiguilles 210 vérifiant les caractéristiques précédentes. Plus spécifiquement, chaque groupe de microaiguilles 210 comprend au moins une microaiguille 210 fonctionnalisée, c'est-à-dire destinée à une même fonction. On distingue ainsi généralement un groupe formant une électrode de travail, un groupe formant une électrode de référence, et éventuellement un groupe formant une contre-électrode et parfois un groupe formant une électrode neutre (qui se distingue en matériau de l'électrode de référence).

Les microaiguilles 210 consistent avantageusement en un réseau de microaiguilles 210 au contact de la peau lorsque la capsule 220 est placée sur le corps d'une personne.

Afin d'éviter les contaminations chimiques entre différents groupes de microaiguilles 210, notamment par fabrication de peroxyde d'hydrogène lors de la réaction biochimique entre la microaiguille et l'analyte, les groupes sont espacées d'au moins 1mm, voire 2mm ou 3 mm. Cela signifie que la distance entre deux microaiguilles 210 de deux groupes respectifs est supérieure ou égale à 1mm, voire 2mm ou 3 mm.

Le capteur présenté est avantageusement monté sur une capsule 220 qui peut se coupler à un boitier 120 pour former un système de surveillance corporelle.

La fin de la description vise à présenter cela.

### Architecture générale

En référence à la **figure 1****,** la présente invention concerne un système électronique 1 de surveillance corporelle. Il s'agit d'une amélioration du dispositif du document WO2018104647. Par conséquent, l'invention se place dans le même concept général de système intégral autonome à faible douleur, faible risque hygiénique et réutilisable.

Par surveillance corporelle, on entend la vérification de constantes biochimiques d'une personne porteuse du système 1, typiquement la concentration en une protéine, une hormone, un marqueur, en oxygène, en nutriments, etc., dans le liquide interstitiel de la personne. On citera l'exemple de la glycémie. L'homme du métier pourra surveiller si besoin d'autres grandeurs physiques corporelles telles que le lactate, l'hydratation, etc.

La description sera illustrée avec du liquide interstitiel mais s'applique aux autres liquides corporels tels que le sang.

Le système 1 est dit autonome, car il ne nécessite pas l'utilisation de matériel supplémentaire.

Le système 1 est destiné à être attaché à un membre d'un être vivant, typiquement un bras ou une jambe d'un être humain. La zone d'attache privilégiée est le poignet où le système 1 s'apparente à une montre.

Le système 1 est formé de deux modules 100, 200 reliées entre eux par une liaison séparable 300 qui est réutilisable.

Le premier module 100 comprend notamment des moyens d'attache et de serrage 110 (qui peut comprendre un bracelet ou une lanière 112 et un réceptacle 114) du système 1 à un membre et le deuxième module 200 comprend une capsule 220 qui intègre le capteur décrit précédemment **(figure 3**). Les microaiguilles 210 du capteur, lorsque le premier module 100 est en position sur le membre, permettent d'analyser un fluide corporel, comme mentionné précédemment.

Comme mentionné précédemment, les microaiguilles 210 consistent avantageusement en un réseau de microaiguilles 210 au contact de la peau lorsque le premier module 100 est placé sur le corps d'une personne.

De façon préférée, lesdits microaiguilles 210 comprennent entre quatre et cinquante microaiguilles, sensiblement pyramidales.

Les deux modules 100 et 200 présentent chacun une face de couplage 122, 222, de forme complémentaire, qui permet de placer le deuxième module 200 dans un emplacement d'accueil du premier module 100

Enfin, le premier module 100 et le deuxième module 200 sont configurés pour être couplés par une liaison séparable 300. Cette liaison 300 solidarise les deux modules 100, 200.

Comme illustré sur la **figure 1****,** le premier module 100 comprend en outre un boitier 120 dans lequel sont disposés des moyens de traitement de données (en particulier un processeur ou un microcontrôleur) configurés pour traiter des mesures acquises par le capteur, et le cas échéant des moyens de stockage de données (notamment une mémoire, en particulier de type flash, et/ou la mémoire du microcontrôleur) permettant par exemple de stocker ces mesures, et/ou une date de la première utilisation de chaque capteur pour calculer une date de péremption du ou des capteurs (les capteurs biochimiques ont une durée de vie limitée). Les moyens de traitement de données servent aussi à générer des consignes vers différentes composants. Dans le cadre de cette description, ces différentes fonctions sont assurées par une même unité. Toutefois, il est possible de prévoir des processeurs dédiés. Le système comprend également une batterie pour l'alimentation électrique des composants, avantageusement rechargeable, par exemple via un port (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran, éventuellement tactile pour afficher les résultats de la surveillance à l'utilisateur.

L'homme du métier connait des algorithmes de traitement de mesures de capteurs 24 et des interfaces associées, et saura les implémenter dans le présent système 1.

Le boitier 120 comprend en outre des connecteurs électriques, sur sa face de couplage 122 avec la face de couplage 222 capsule 220.

La capsule 220 du deuxième module a une forme de boite fermée, typiquement étanche, qui peut se coupler avec le boitier 120 100. Cette capsule 220 est interchangeable, ce qui permet d'obtenir un système économique et efficace, où seules les parties dites consommables doivent être changées. La capsule 220 peut avoir une forme annulaire, avec une ouverture traversante 224 au centre. La capsule 220 comprend des connecteurs électriques 226, sur une face de couplage 222 avec le boitier 120, qui peuvent coopèrent avec les connecteurs électriques du boitier 120. S'il y a transmission de fluide depuis le deuxième module vers le premier alors des connecteurs fluidiques complémentaires sont prévus sur la capsule 220 et le boitier 120.

Enfin, le deuxième module 200 comprend un patch 250, solidaire de façon amovible à la capsule 220.

Le deuxième module (capsule 220 et patch 250) forme un ensemble interchangeable du système qui est choisi selon le type de surveillance voulu et en fonction de l'état de détérioration des microaiguilles 210 et/ou du capteur.

En effet, dans la mesure où la capsule 220 contient les microaiguilles 210 et/ou le capteur, changer de capsule 220 permet de changer de capteurs si ceux-ci sont en fin de vie ou si l'on souhaite changer de grandeur physique mesurée, en une manipulation simple, rapide et sure, sans devoir jeter d'autres parties (en particulier le premier module).

Et dans la mesure où la capsule 220 minimise la quantité d'élément et/ou matériaux coûteux (équipement électronique avancé tel qu'une batterie ou des moyens de communication sans fil), elle est relativement peu chère.

## Revendications

1. Capteur pour mesurer des analytes présentes dans un fluide corporel dans un être vivant, le capteur comprend :
- un substrat (242),
- au moins une micro-aiguille (210), la micro-aiguille (210) comprenant :
une base (210a), solidaire du substrat (242), et comprenant en partie un revêtement métallique en forme de piste (210c),
une pointe (210b), solidaire de la base (210a), entièrement recouverte d'un revêtement métallique,
un élément biochimique recouvrant la pointe (210b), apte à réagir avec des analytes et qui recouvre au moins partiellement le revêtement métallique de la microaiguille (210),
**caractérisé en ce que** le ratio surface métallique de la base (210a) sur surface totale de la base (210a) est compris entre 1 et 20%.

2. Capteur selon la revendication 1 dans lequel la hauteur de la base (210a) est supérieure à 140 µm, préférablement supérieure à 200µm.

3. Capteur selon l'une quelconque des revendications 1 à 2, comprenant une pluralité de groupes comprenant au moins une microaiguille (210) telle que définie dans la revendication 1, un groupe définissant une électrode de travail, un groupe définissant une électrode de référence, et préférablement une contre-électrode, voire une électrode neutre.

4. Capteur selon la revendication 3 dans lequel les microaiguilles des deux groupes sont écartées entre elles d'au moins 1 mm.

5. Capteur selon l'une quelconque des revendications 1 à 3, dans lequel la hauteur de la pointe (210b) est comprise entre 200 et 700 µm, préférablement entre 300 et 500 µm.

6. Capteur selon l'une quelconque des revendications 1 à 5, dans lequel la base (210a) a une forme pyramidale.

7. Capteur selon l'une quelconque des revendications 1 à 6, dans lequel le substrat est en polymère ou silicium, ou pas en métal.

8. Capteur selon l'une quelconque des revendications 1 à 7, dans lequel l'élément biochimique recouvre intégralement le revêtement métallique de la pointe (210a) et partiellement le revêtement métallique de la base (210a).

9. Système de surveillance corporelle (1) destiné à être attaché à un membre d'un être vivant, comprenant :
- un boitier (120), comprenant une face de couplage (122),
- une capsule (220) comprenant un capteur selon l'une quelconque des revendications 1 à 8, la capsule pouvant s'engager de façon amovible avec la face de couplage (122) du boitier (120).

10. Système de surveillance corporelle (1) selon la revendication 9, comprenant en outre un patch (250) attachable à la capsule (220), le patch (250) ayant un pouvoir adhésif à la peau.
